# FASCICULE DE BREVET EUROPEEN

(11) **EP 0 932 665 B1**
(45) Date de publication et mention de la délivrance du brevet: **27.08.2008**
(21) Numéro de dépôt: 97938964.0
(22) Date de dépôt: 29.08.1997
(51) Int. Cl.: C12N 9/10, C12N 15/54, C12Q 1/68, G01N 33/50

(54) **PROCEDE DE TRI DE MOLECULES ANTIFONGIQUES ACTIVES SUR L'ACTIVITE GLUCANOSYLTRANSFERASE**
METHODE ZUR IDENTIFIKATION ANTIFUNGISCHER MOLEKÜLE, WELCHE AUF DIE GLUKANOSYLTRANSFERASEAKTIVITÄT WIRKEN
METHOD FOR SORTING ANTIFUNGAL MOLECULES ACTING ON THE GLUCANOSYLTRANSFERASE ACTIVITY

(30) Priorité: 30.08.1996 US 24910 P
(43) Date de publication de la demande: 04.08.1999
(73) Titulaire: INSTITUT PASTEUR, 75724 Paris Cédex 15 (FR)
(72) Inventeur: FONTAINE, Thierry, F-92130 Issy-les-Moulineaux (FR); HARTLAND, Robbert, Willetton, W.A. 6155 (AU); MOUYNA, Isabelle, F-75012 Paris (FR); LATGE, Jean-Paul, F-92130 Issy-les-Moulineaux (FR)
(74) Mandataire: Phélip, Bruno
(86) Numéro de dépôt international: PCT/FR1997/001540
(87) Numéro de publication internationale: WO 1998/008937

(56) Documents cités:
- NUOFFER, C. ET AL.: "Determinants for glycophospholipid anchoring of the Saccharomyces cerevisiae GAS1 protein to the plasma membrane" MOL. CELL. BIOL. , vol. 11, no. 1, 1991, pages 27-37, XP002052557 cité dans la demande
- SAPORITO-IRWIN, S. M. ET AL.: "PHR1, a pH-regulated gene of candida albicans, is required for morphogenesis" MOL. CELL. BIOL. , vol. 15, no. 2, 1995, pages 601-613, XP002052558 cité dans la demande
- HARTLAND R.P. ET AL.: "A secreted beta-glucan-branching enzyme from Candida albicans" PROC. R. SOC. LOND. B, vol. 246, 1991, pages 155-160, XP002052559
- HARTLAND, R.P. ET AL.: "A novel beta-(1-3)-Glucanosyltransferase from the cell wall of Aspergillus fumigatus" J. BIOL. CHEM., vol. 271, no. 43, 25 octobre 1996, pages 26843-49, XP002052560
- RAD. R.M. ET AL.: "Analysis of the DNA sequence ..." YEAST, vol. 13, 1997, pages 281-286, XP002052561 & DATABASE EMBL Accession number Z74772, juillet 1996 HABBIG, B. ET AL:

### LISTE DE SEQUENCES

(1) INFORMATIONS GENERALES:
   (i) DEPOSANT:
      (A) NOM: INSTITUT PASTEUR
      (B) RUE: 25 RUE DU DOCTEUR ROUX
      (C) VILLE: PARIS
      (E) PAYS: FRANCE
      (F) CODE POSTAL: 75724
   (ii) TITRE DE L' INVENTION: BETA(1-3)GLUCANOSYLTRANSFERASE
   (iii) NOMBRE DE SEQUENCES: 12
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 1359 paires de bases
      (B) TYPE: nuclotide
      (C) NOMBRE DE BRINS: simple
      (D) CONFIGURATION: linaire
   (ii) TYPE DE MOLECULE: ADN (gnomique)
   (ix) CARACTERISTIQUE:
      (A) NOM/CLE: CDS
      (B) EMPLACEMENT:1..1357
   (xi) DESCRIPTION DE LA SEQUENCE: SEQ ID NO: 1:
   ATG AAG GCC TCT GCT GTT ACT GCC GCT CTC GCC GTC GGT GCT TCC ACC      48
   Met Lys Ala Ser Ala Val Thr Ala Ala Leu Ala Val Gly Ala Ser Thr
    1               5                  10                  15
   GTT CTG GCA GCC CCC TCC ATC AAG GCT CGT GAC GAC GTT ACT CCC ATC      96
   Val Leu Ala Ala Pro Ser Ile Lys Ala Arg Asp Asp Val Thr Pro Ile
               20                  25                  30
   ACT GTC AAG GGC AAT GCC TTC TTC AAG GGC GAT GAG CGT TTC TAT ATT     144
   Thr Val Lys Gly Asn Ala Phe Phe Lys Gly Asp Glu Arg Phe Tyr Ile
           35                  40                  45
   CGC GGT GTC GAC TAC CAG CCC GGT GGC TCC TCC GAC CTG GCT GAT CCC     192
   Arg Gly Val Asp Tyr Gln Pro Gly Gly Ser Ser Asp Leu Ala Asp Pro
       50                  55                  60
   ATC GCT GAT GCC GAT GGT TGC AAG CGT GAC ATT GCC AAG TTC AAG GAG     240
   Ile Ala Asp Ala Asp Gly Cys Lys Arg Asp Ile Ala Lys Phe Lys Glu
    65                  70                  75                  80
   CTG GGC CTG AAC ACT ATC CGT GTC TAC TCG GTC GAC AAC TCC AAG AAC     288
   Leu Gly Leu Asn Thr Ile Arg Val Tyr Ser Val Asp Asn Ser Lys Asn
                    85                  90                  95
   CAC GAT GAG TGT ATG AAT ACA CTG GCT GAT GCT GGC ATC TAT CTG GTG     336
   His Asp Glu Cys Met Asn Thr Leu Ala Asp Ala Gly Ile Tyr Leu Val
               100                 105                 110
   CTC GAT GTC AAC ACT CCC AAG TAC TCC ATC AAC CGC GCC AAG CCT AAG     384
   Leu Asp Val Asn Thr Pro Lys Tyr Ser Ile Asn Arg Ala Lys Pro Lys
           115                 120                 125
   GAG TCG TAC AAC GAT GTC TAC CTC CAG TAT ATC TTC GCT ACC GTT GAT     432
   Glu Ser Tyr Asn Asp Val Tyr Leu Gln Tyr Ile Phe Ala Thr Val Asp
       130                 135                 140
   GCT TTC GCC GGT TAC AAG AAC ACC CTC GCT TTC TTC TCC GGC AAC GAG     480
   Ala Phe Ala Gly Tyr Lys Asn Thr Leu Ala Phe Phe Ser Gly Asn Glu
   145                 150                 155                 160
   GTT ATC AAC GAT GGC CCT TCC TCC TCT GCT GCT CCC TAC GTC AAG GCC     528
   Val Ile Asn Asp Gly Pro Ser Ser Ser Ala Ala Pro Tyr Val Lys Ala
                   165                 170                 175
   GTC ACT CGT GAT CTG CGT CAG TAC ATC CGT AGC CGC AAG TAC CGT GAG     576
   Val Thr Arg Asp Leu Arg Gln Tyr Ile Arg Ser Arg Lys Tyr Arg Glu
               180                 185                 190
   ATT CCT GTC GGC TAC TCG GCT GCC GAT ATC GAC ACC AAC CGT CTT CAG     624
   Ile Pro Val Gly Tyr Ser Ala Ala Asp Ile Asp Thr Asn Arg Leu Gln
           195                 200                 205
   ATG GCC CAG TAT ATG AAC TGC GGT TCC GAC GAC GAG CGC AGT GAC TTC     672
   Met Ala Gln Tyr Met Asn Cys Gly Ser Asp Asp Glu Arg Ser Asp Phe
       210                 215                 220
   TTC GCT TTC AAC GAC TAC TCC TGG TGC GAT CCC TCC TCT TTC AAA ACC     720
   Phe Ala Phe Asn Asp Tyr Ser Trp Cys Asp Pro Ser Ser Phe Lys Thr
   225                 230                 235                 240
   TCG GGC TGG GAT CAG AAG GTC AAG AAC TTC ACT GGC TAC GGT CTT CCT     768
   Ser Gly Trp Asp Gln Lys Val Lys Asn Phe Thr Gly Tyr Gly Leu Pro
                   245                 250                 255
   CTC TTC CTG TCC GAA TAC GGC TGC AAC ACC AAC AAG CGT CAA TTC CAA       816
   Leu Phe Leu Ser Glu Tyr Gly Cys Asn Thr Asn Lys Arg Gln Phe Gln
               260                 265                 270
   GAA GTC AGC TCT CTC TAC TCC ACG GAC ATG ACT GGT GTC TAC TCT GGT       864
   Glu Val Ser Ser Leu Tyr Ser Thr Asp Met Thr Gly Val Tyr Ser Gly
           275                 280                 285
   GGT CTC GTG TAC GAG TAC TCT CAG GAG GCC AGC AAC TAC GGT CTG GTG       912
   Gly Leu Val Tyr Glu Tyr Ser Gln Glu Ala Ser Asn Tyr Gly Leu Val
       290                 295                 300
   GAG ATT AGC GGC AAC AAT GTC AAG GAG CTC CCA GAC TTC GAC GCT CTG       960
   Glu Ile Ser Gly Asn Asn Val Lys Glu Leu Pro Asp Phe Asp Ala Leu
   305                 310                 315                 320
   AAG ACC GCG TTC GAA AAG ACC TCC AAC CCC TCC GGC GAC GGC AAC TAC      1008
   Lys Thr Ala Phe Glu Lys Thr Ser Asn Pro Ser Gly Asp Gly Asn Tyr
                   325                 330                 335
   AAC AAG ACT GGT GGT GCC AAC CCT TGC CCC GCT AAG GAC GCT CCC AAC      1056
   Asn Lys Thr Gly Gly Ala Asn Pro Cys Pro Ala Lys Asp Ala Pro Asn
               340                 345                 350
   TGG GAC GTT GAC AAC GAT GCT CTT CCT GCC ATC CCC GAG CCC GCC AAG      1104
   Trp Asp Val Asp Asn Asp Ala Leu Pro Ala Ile Pro Glu Pro Ala Lys
           355                 360                 365
   AAG TAC ATG ACT GAG GGT GCT GGC AAG GGC CCT GGT TTT GCC GGA CCT      1152
   Lys Tyr Met Thr Glu Gly Ala Gly Lys Gly Pro Gly Phe Ala Gly Pro
       370                 375                 380
   GGC AGC CAG GAC CGT GGT ACC CAG TCC ACT GCC ACT GCT GAG CCC GGA      1200
   Gly Ser Gln Asp Arg Gly Thr Gln Ser Thr Ala Thr Ala Glu Pro Gly
   385                 390                 395                 400
   TCT GGC TCT GCC ACT GGA AGC AGC AGC AGC GGC ACC TCC ACC TCT TCC      1248
   Ser Gly Ser Ala Thr Gly Ser Ser Ser Ser Gly Thr Ser Thr Ser Ser
                   405                 410                 415
   AAG GGC GCT GCA GCT GGC CTG ACT GTC CCT AGC CTG ACC ATG GCT CCC      1296
   Lys Gly Ala Ala Ala Gly Leu Thr Val Pro Ser Leu Thr Met Ala Pro
               420                 425                 430
   GTT GTC GTT GGT GCG GTT ACA CTC CTG TCC ACC GTC TTC GGC GCT GGC      1344
   Val Val Val Gly Ala Val Thr Leu Leu Ser Thr Val Phe Gly Ala Gly
           435                 440                 445
   CTC GTC CTC TTG TGA                                                  1359
   Leu Val Leu Leu
       450
(2) INFORMATIONS POUR LA SEQ ID NO: 2:
   (i) CARACTERISTIQUES DE LA SEQUENCE:
      (A) LONGUEUR: 452 acides amins
      (B) TYPE: acide amin
      (D) CONFIGURATION: linaire
   (ii) TYPE DE MOLECULE: protine
   (xi) DESCRIPTION DE LA SEQUENCE: SEQ ID NO: 2:
(2) INFORMATIONS POUR LA SEQ ID NO:3:
   (i) CARACTERISTIQUES DE LA SEQUENCE:
      (A) LONGUEUR: 426 acides amins
      (B) TYPE: acide amin
      (C) NOMBRE DE BRINS:
      (D) CONFIGURATION: linaire
   (ii) TYPE DE MOLECULE: protine
   (ix) DESCRIPTION DE LA SEQUENCE: SEQ ID NO: 3:
(2) INFORMATIONS POUR LA SEQ ID NO: 4:
   (i) CARACTERISTIQUES DE LA SEQUENCE:
      (A) LONGUEUR: 38 paires de bases
      (B) TYPE: nuclotide
      (C) NOMBRE DE BRINS: simple
      (D) CONFIGURATION: linaire
   (ii) TYPE DE MOLECULE: ADN (gnomique)
   (xi) DESCRIPTION DE LA SEQUENCE: SEQ ID NO: 7:
      GSYTTCTTCK CYGGCAACGA GGTT 24
(2) INFORMATIONS POUR LA SEQ ID NO: 8:
   (i) CARACTERISTIQUES DE LA SEQUENCE:
      (A) LONGUEUR: 24 paires de bases
      (B) TYPE: nuclotide
      (C) NOMBRE DE BRINS: simple
      (D) CONFIGURATION: linaire
   (ii) TYPE DE MOLECULE: ADN (gnomique)
   (xi) DESCRIPTION DE LA SEQUENCE: SEQ ID NO: 8:
      GTTGCAGCCG WATTCGGASA YGAA 24
(2) INFORMATION POUR LA SEQ ID NO: 9:
   (i) CARACTERISTIQUES DE LA SEQUENCE:
      (A) LONGUEUR: 394 paires de bases
      (B) TYPE: nuclotide
      (C) NOMBRE DE BRINS: simple
      (D) CONFIGURATION: linaire
   (ii) TYPE DE MOLECULE: ADN (gnomique)
   (xi) DESCRIPTION DE LA SEQUENCE: SEQ ID NO: 9:
   GGCTTCTTCG CCGGCAACGA GGTTATCAAC GAGCAGAGTG TCAAGAACGT TCCCACTTAC     60
   GTCCGGGTAT GTCATCCATC CCCACAGCTT ACGATTGCCT GTCCACTGAC ACTCTCGTAG    120
   GCGACTCAGC GTGACATGAA GGACTACATC GCAAAGAACC TTGACCGCAG CATTCCTGTT    180
   GGCTATTCTG CTGCCGATAT TCGTCCCATC CTCATGGACA CCCTCAACTA CTTCATGTGC    240
   GCTGACGATG CTAATTCCCA ATCGGACTTC TTCGGCCTCA ACTCCTACTC GTGGTGCGGC    300
   AACTCGTCCT ACACCAAGAG TGGCTACGAT GTCCTCACCA AGGACTTTGC CGACGCCTCT    360
   ATCCCCGTCT TCATCTCCGA ATTCGGCTGC AACA                                394
(2) INFORMATIONS POUR LA SEQ ID NO: 10:
   (i) CARACTERISTIQUES DE LA SEQUENCE:
      (A) LONGUEUR: 36 acides amins
      (B) TYPE: acide amin
      (C) NOMBRE DE BRINS:
      (D) CONFIGURATION: linéaire
   (ii) TYPE DE MOLECULE: protine
   (xi) DESCRIPTION DE LA SEQUENCE: SEQ ID NO: 10:
   Gly Phe Phe Ala Gly Asn Glu Val Ile Asn Glu Gln Ser Val Lys Asn
   1               5                   10                  15
   Val Pro Thr Tyr Val Arg Val Cys His Pro Ser Pro Gln Leu Thr Ile
               20                  25                  30
   Ala Cys Pro Leu
           35
(2) INFORMATIONS POUR LA SEQ ID NO: 11:
   (i) CARACTERISTIQUES DE LA SEQUENCE:
      (A) LONGUEUR: 339 paires de bases
      (B) TYPE: nuclotide
      (C) NOMBRE DE BRINS: simple
      (D) CONFIGURATION: linaire
   (ii) TYPE DE MOLECULE: ADN (gnomique)
   DESCRIPTION DE LA SEQENCE: SEQ ID NO: 11:
   (i) CARACTERISTIQUES DE LA SEQUENCE:
      (A) LONGUEUR: 113 acides amins
      (B) TYPE: acide amin
      (C) NOMBRE DE BRINS:
      (D) CONFIGURATION: linaire
   (ii) TYPE DE MOLECULE: protine
   (xi) DESCRIPTION DE LA SEQUENCE: SEQ ID NO: 12:

## Description

La présente invention concerne une protéine ayant une activité de type glucanosyltransférase, et plus particulièrement une activité de type β-(1-3) glucanosyltransférase.

La présente invention décrit également des oligonucléotides codant pour cette protéine ayant une activité enzymatique.

De même elle décrit des molécules ayant un effet sur l'activité de cette enzyme.

Les infections opportunistes fongiques dûes à *Candida, Aspergillus. Cryptococcus* et *Pneumocystis* sont responsables de la croissance de la morbidité et de la mortalité parmi les patients atteints du SIDA et autres patients présentant une immunité compromise cliniquement. De plus, la levure *Candida* et les dermatophytes demeurent aujourd'hui un problème médical important parmi les patients présentant une immunité suffisante. Malgré l'augmentation du nombre des infections dues aux champignons pathogènes et opportunistes, la thérapie contre les mycoses ne s'est pas améliorée durant ces dernières années. Deux familles de médicaments sont utilisés: les azoles et l' Amphotéricine B. Ces médicaments présentent certains inconvénients puisque le traitement à base d'Amphotéricine B est associé à une néphrotoxicité et celui à base d'azole est plus fongistatique que fongicide.

Les champignons sont des micro-organismes de type eucaryote qui partagent la plupart des voies biochimiques avec leur hôte avec une exception importante : la biosynthèse de la paroi cellulaire. La paroi cellulaire est une enveloppe rigide qui protège la cellule contre l'environnement et les stress mécaniques, mais également une structure dynamique qui est impliquée dans le transport des ions et des macromolécules et dans la localisation d'enzymes impliquées dans la croissance fongique. Par conséquent, la désorganisation de l'organisation de la paroi cellulaire doit être préjudiciable aux champignons.

Le squelette de la paroi cellulaire fongique est principalement constituée de polymères de type polysaccharide (β (1-3) glucanes, mannanes, chitine) que l'on ne trouve pas chez les humains. Pour cette raison, la biosynthèse de la paroi cellulaire a été une cible pour la recherche de nouveaux médicaments antifongiques. Les pénicilllines et les céphalosporines qui sont tous deux des inhibiteurs de la paroi cellulaire bactérienne et des antibiotiques potentiels apportent une crédibilité à cette hypothèse. De plus, beaucoup de molécules qui inhibent le développement de la paroi cellulaire fongique ont des propriétés antifongiques (Debono et Gordee, 1994, Annu. Rev. Microbiol, 48, 471-497). Parmi eux, on peut citer:
1) Les familles des lipopeptides des échinocandins et des glycopeptides des palulacandins qui sont des inhibiteurs non compétitifs du complexe de la glucane synthétase.
2) Les polyoxines et les nikkomycines qui sont des analogues de l'UDP-GlcNac et des inhibiteurs compétitifs potentiels de la chitine synthétase, et
3) Les pradimicines liant le mannane et les bénanomicines.

La synthèse du β (1-3) glucane et de la chitine est sous le contrôle de complexes d'enzymes (Glucane synthétase et chitine synthétase) qui sont localisés au niveau de la membrane plasmique. Une fois que les polymères sont relargés dans l'espace périplasmique, des liaisons réticulantes s'effectuent entre les polymères et sont responsables de la rigidité de la paroi cellulaire. Les protéines et les gènes des glucane et chitine synthétases commencent à être plutôt bien compris.

Mais l'inhibition des chitine et glucane synthétases pour une molécule nécessite trois étapes: le transport à travers la paroi cellulaire, la traversée de la membrane plasmique et le transport à l'intérieur de la cellule vers la cible, chaque étape pouvant être responsable de l'échec de l'inhibiteur enzymatique à être un médicament antifongique efficace de même que de la sélection de résistants contre le médicament.

Les transférases qui sont responsables de l'établissement des liaisons covalentes entre les différents polymères de la paroi ont été très peu étudiées jusqu'à maintenant.

Ces enzymes représentent une meilleure cible que les complexes de chitine et glucane synthétases puisqu'elles sont d'un accès plus facile pour un médicament antifongique putatif.

Nuoffer et al. (1991, Mol. Cell. Bio., 11, 27-37) ont décrit une glycoprotéine, appelée Gas1p, exposée à la surface de la levure *Saccharomyces cerevisae.* Les gènes codant pour cette protéine ont été clonés. La fonction de la protéine Gas1p n'est pas essentielle pour la viabilité de la cellule, et n'a pas été déterminée.

Saporito-Irwing et al (1995, Mol. Cell. Biol., 15, 601-613) ont isolé un gène provenant de la levure *Candida albicans,* désigné PHR1. La séquence d'acides aminés déterminé de la protéine PHR1 était identique de 56% à celle de la protéine Gas 1. Le gène est régulé en réponse au pH du milieu de culture. De même que pour la protéine gas1p, aucune fonction n'a été +déterminée. Hartland et al (1991, Proc. R. Soc. Lond., 246, 155-160) out isolé one β-(1,3) glucanosyltransferase chez Candida albicans.

Il ressort clairement de cette analyse de l'art antérieur qu'il existait un problème résidant dans l'obtention de molécules ayant une activité antifongique efficace.

Les inventeurs ont résolu ce problème.

Ils ont montré que l'introduction de mutations dans une glucanosyltransférase provenant d'Aspergillus fumigatus interfère avec le développement de ce micro-organisme.

Ils ont de plus déterminés la séquence de plusieurs de ces enzymes.

La présente invention concerne de ce fait une protéine ayant une activité de type β-(1-3)-glucanosyltransférase caractérisée en ce qu'elle présente au moins 85% d'homologie avec la protéine ayant une des séquences SEQ ID N°2 ou SEQ ID N°:3 suivantes :

De préférence, cette protéine présente un poids moléculaire d'environ 44 kD, ou d'environ 49 kD si elle porte au moins un radical de type N-glycosyle.

La présente invention décrit de plus des séquences nucléotidiques codant pour la proteine telle que décrit ci-dessus, et plus particulièrement des séquences d'ADN (ADNc ou ADN génomique) ou des séquences d'ARN.

Une telle séquence d'ADN peut être celle présentant une homologie d'au moins 50%, de préférence 60%, et encore plus préférentiellement de 85% avec la séquence génomique SEQ ID N° 1, ou une partie de la séquence suivante

Cette séquence a été incluse dans un fragment de 2,2 kb, qui a lui-même été inclus dans le site X baI du vecteur pUC 19 (Maniatis et al, 1989, Cold Spring Harbor Laboratoires Press). La souche E.coli DH₅α portant le vecteur modifié de cette façon, a été déposée auprès de la Collection Nationale de Culture de Micro-organismes de l'Institut Pasteur (CNCM) le 26 juillet 1996 sous le numéro I-1763.

Une telle séquence peut également être celle présentant une homologie d'au moins 50%, de préférence 60%, et encore plus préférentiellement de 85% avec la séquence d'ADN complémentaire comprise dans un fragment de 1,4 kb qui a été inclus dans le vecteur pCRII (In Vitrogen). L'ensemble, porté par la souche E.coli DH₅α, a été déposé auprès de la Collection Nationale de Culture de Micro-organismes de l'Institut Pasteur (CNCM) le 26 juillet 1996 sous le numéro I-1762.

Ces deux souches sont des objets de la présente invention.

La présente invention a en outre pour objet une méthode pour la détection de mutations dans la séquence SEQ ID N°1, , dans un échantillon biologique contenant des séquences nucléotidiques, comprenant les étapes suivantes :
a) mise en contact de l'échantillon biologique avec les amorces nucléotidiques P3 et P4 présentant respectivement les séquences SEQ ID N°7 et SEQ ID N°8 suivantes :
   SEQ ID NO:7:
      GSYTTCTTCK CYGGCAACGA GGTT
   SEQ ID NO: 8:
      GTTGCAGCCG WATTCGGASA YGAA

les séquences nucléotidiques contenues dans l'échantillon ayant été éventuellement mises sous une forme permettant leur hybridation dans des conditions permettant l'hybridation des amorces avec les séquences nucléotidiques.
b) amplification de séquences nucléotidiques
c) mise en évidence des produits d'amplification, et
d) détection des mutations par des techniques appropriées

Les protéines selon l'invention peuvent être obtenues par purification à partir d'un autolysat d*'Aspergillus fumigatus*. La protéine peut être purifiée par quatre étapes de chromatographie d'échange d'ions et une étape de filtration sur gel.

Lesdites protéines peuvent également être obtenues par des méthodes du génie génésique. Par exemple, la séquence SEQ ID N° 1, privée si possible de sa partie C terminale, peut être clonée dans un vecteur approprié, et être exprimée dans un système d'expression, tel que le système de *Pichia pastoris,* commercialisé par In Vitrogen.

Dans ce système la séquence du gène codant pour la protéine est clonée dans un vecteur d'expression, puis linéarisé. Des protoplastes provenant de P. *pastoris* sont transformés avec le vecteur linéarisé.

Les clones, dans lesquels une recombinaison s'effectue et permet le remplacement de la séquence aox1 par la séquence du gène de la protéine que l'on cherche à produire, sont choisis pour leur capacité à pousser sur un milieu déficient en histidine. L'home de l'art peut se référer au « Manual of methods for expression of recombinant proteins in *Pichia pastoris* », édité par In Vitrogen.

La protéine, exprimée de cette façon, sécrétée si possible dans le milieu de culture, est récupérée par des procédés connus par l'homme de l'art.

Une telle protéine peut être utilisée, en particulier pour le criblage de molécules pour identifier leur activité antifongique.

Ainsi, un autre objet de la présente invention est un procédé pour la detection de l'activité antiforgique de molécules, par inhibition de l'activité de la β-(1,3) glcanosyltransférase, comprenant les étapes suivantes:
- mettre en présence la molécule à détecter et la protéine telle que décrite ci-dessus, ou codée par la séquence SEQ ID N°1 , et
- déterminer l'effet inhibiteur d'une telle molécule.
sur ladite protéine en mesurant l'activité de la p- (1-3) glucanosyltansférase (BGT2), de ladite proteine. Une telle activité peut être déterminée en mettant en présence ladite protéine avec un substrat sur lequel elle a un effet, qui peut être composé de laminarioligosaccharides comprenant au moins 10 radicaux glucosyle liés par des liaisons β- (1-3). En fait, lorsque la protéine est active, elle clive une partie de la molécule et lie le fragment obtenu sur l'extrémité non réductrice d'une molécule substrat non clivée.

Le produit, résultant de l'activité de la protéine est sous forme de produits de couplage de deux laminorioligosaccharides. Ce produit peut être détecté par tout procédé permettant la séparation des oligosaccharides ayant des degrés différents de polymérisation, en particulier par des méthodes de chromatographie, telles que la chromatographie liquide à haute pression (HPLC) ou la chromatographie en couche mince (CCM). Cette méthode, bien que moins précise que la première, est la méthode la plus facile à mettre en oeuvre.

Pour la mise en oeuvre de ces méthodes de chromatographie, l'homme de l'art peut se référer au manuel suivant: Carbohydrate analysis : a practical approach. Chaplin et Kennedy, 1986, IRC Press of Oxford.

Cette méthode de détection permet de déterminer si les molécules détectées possèdent une activité antifongique.

Ces molécules ayant une activité antifongique présentent des effets sur l'activité de la β-(1-3)glucanosyltransférase desdites protéines. Ces effets peuvent être par exemple l'inhibition de cette activité.

La présente invention est illustrée, sans en être limitée, par les exemples suivants:
La figure 1 illustre une analyse de type SDS-PAGE de la protéine purifiée de 49 kDa: ligne a, standards des poids moléculaires; ligne b, protéine purifiée de 49 kDa (1,5 µg); ligne c, protéine purifiée de 49 kDa (1,5 µg) après traitement avec de la N-glycosidase F, ligne d, N-glycosidase seulement. Le poids moléculaire des bandes des protéines et de la N-glycosidase F sont indiqués.
La figure 2 représente l'analyse de type HPAEC des produits provenant de l'incubation de l'enzyme de 49 kDa avec des laminarioligosaccharides réduits. On a incubé la protéine purifiée de 49 kDa avec 8 mM de laminarioligosaccharides réduits de taille G₁₁, G₁₂, G₁₃ ou G₁₄ (laminarioligosaccharides réduits des résidus respectifs 11, 12, 13 ou 14 de glucose) et on a représenté les profils HPAEC provenant d'échantillons aux temps zéro et 15 min, en indiquant la taille des produits principaux.
La figure 3 représente une analyse de type HPAEC de produits provenant de l'incubation de l'enzyme de 49 kDa avec 8 mM de rG₁₆. On a représenté les profils HPAEC provenant d'échantillons pris aux temps zéro, 30 min et 120 min, en indiquant la taille des produits principaux.
La figure 4 illustre l'action de la transférase et des produits formés provenant des laminarioligosaccharides réduits.
La figure 5 illustre l'effet des concentrations variantes des substrats. On a incubé la transférase de 49 kDa avec 3 µM de [³H]-rG₁₁ (1x10⁶ cpm) plus des quantités variantes de rG₁₁ non marqués. On a déterminé le % du transfert en comparant la proportion du marquage formé sous forme de rG₆ et rG₁₆. Le supplément présente les même données avec la concentration des substrats présentée sous forme d'une échelle linéaire.
La figure 6 illustre les effets du pH sur la vitesse du transfert. On a incubé la transférase de 49 kDa avec 8 mM de [³H]-rG₁₁ (1x10⁶ cpm). On a déterminé les vitesses des réactions en mesurant la quantité de marquage formé sous forme de produit rG₁₆. Les tampons utilisés étaient: •, citrate de sodium/acide citrique; o, imidazole/acide citrique; X, acétate de sodium/acide acétique; ■, Tris/glycine, ▲, phosphate/NaOH; □, Tris/acide acétique; Δ, glycine/HCl.
La figure 7 représente la séquence provenant du gène BGT2 de *A.fumigatus* (A) comparée aux gènes PHR1 et GAS1 isolés de *C.albicans* (C) et *S*. *cerevisiae* (S). L'acide aminé surligné correspond au supposé intron.

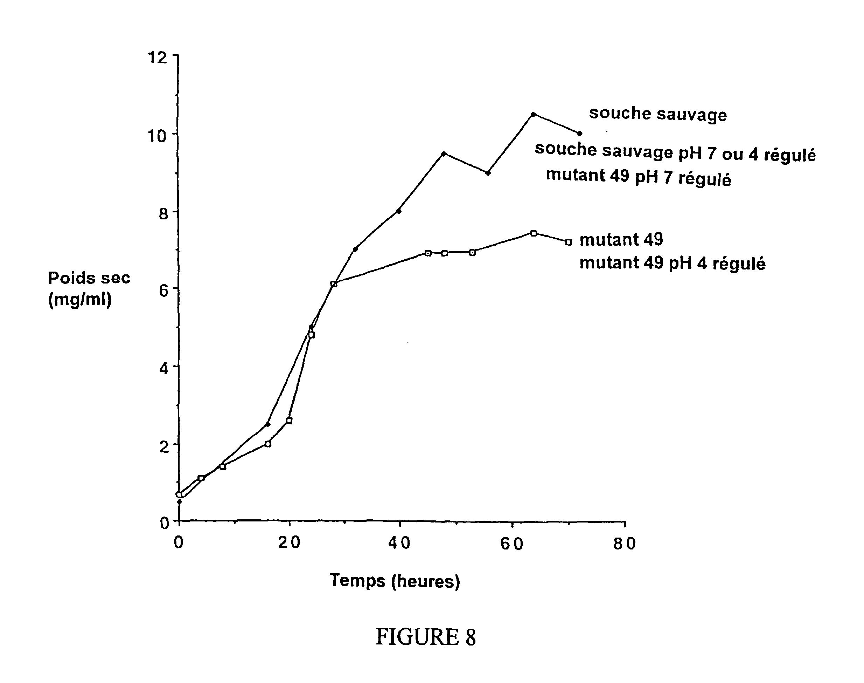

La figure 8 illustre la vitesse de croissance du mutant 49 comparé à la souche sauvage. L'indication d'une valeur de pH signifie que la culture a été faite à un pH régulé (4 ou 7).

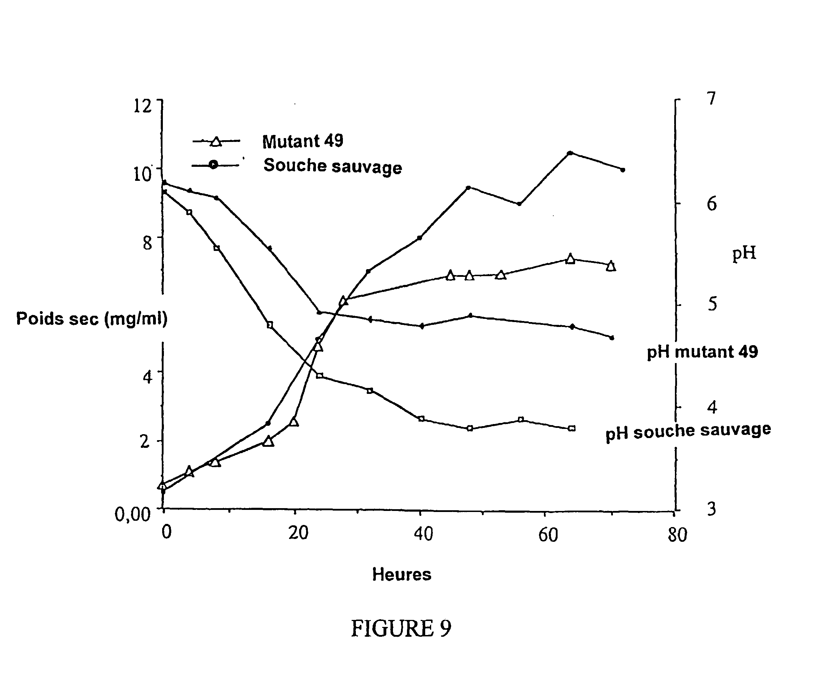

La figure 9 illustre la croissance de la souche sauvage et celle du mutant Δ 49 et l'évolution du pH du milieu de culture au cours de la croissance des deux souches.
La figure 10 est une comparaison des séquences de la protéine de séquence SEQ ID N°2 et des cinq protéines suivantes : Phr 1p, Gas 2p, Gas 3p, Gas 4p et Gas 5p.
Les figures 11 et 12 sont des cartes de restriction des séquences de gènes homologues au gène BGT2, appelés respectivement BGT4 et BGT3.

### EXEMPLES

### PROCEDURES EXPERIMENTALES

### 1. Préparation de la paroi cellulaire et autolyse.

On a fait pousser la souche CBS 144-89 de type *A. fumigatus* (disponible à la Collection Centralbureau voor Schimmelculture) dans un fermenteur de 15 L dans du glucose à 2%, de la mycopeptone à 1% (Biokar Diagnostics) plus du silicone antimousse 426R à 0,1% (Rhodorsil) à 25°C (agitation à 500 tpm, aération à 0,5 vol.vol⁻¹ .min⁻¹) pendant 42 h. On a utilisé en tant qu'inoculum une culture ayant poussée pendant 3 jours dans un fermenteur de 2 L sous les mêmes conditions (8% (v/v)). Les mycélium ont été collectés par filtration sous vide et cassés en les passant à travers un broyeur de type Dyno en présence de billes de verre (W.A. Bachofen AG, Basel, Suisse) (0,5-0,75 mm de diamètre). On a suivi la progression de la dislocation des cellules par examen au microscope. La suspension de mycélium cassée a été centrifugée (8000 g, 15 min) et on a lavé le culot contenant les parois cellulaires 3 fois avec de l'eau et une fois dans de l'acétate de Na 50 mM, Ph 5,6 contenant de l'azide de Na 5 mM avant de le remettre en suspension dans le même tampon (250 g de poids humide par L de tampon) et on l'a incubé (agitation à 200 tpm) à 37°C. Après 72 h, on a centrifugé la suspension (10000g 15 min) et on a placé le surnageant dans une canalisation pour dialyse, on l'a concentré 5 à 10 fois avec du polyéthylèneglycol 20000 dialysé contre de l'acétate de Na 5 mM, pH 5,6, recentrifugé (10000g, 15 min) et filtré (filtre de 0,45 µm). Cette préparation est référencée comme autolysat.

### 2. Purification des enzymes

On a testé des fractions collectées au cours de chaque étape de la chromatographie pour l'activité enzymatique en utilisant le test non radioactif de la transférase (voir ci-dessous). On a appliqué l'autolysat dialysé et concentré sur une colonne (4 x 18 cm) de DEAE-Sepharose Fast-Flow (Pharmacia) équilibrée avec de l'acétate de Na 5 mM, pH 5,6 et on a élué avec un gradient linéaire de NaCl 0 à 1 M (2000 ml) à une vitesse d'écoulement de 240 ml.h⁻¹. On a collecté les fractions contenant l'activité de la transférase, on les a dialysées contre un tampon contenant du β-mercaptoéthanol 10 mM, de l'EDTA 5 mM, de l'acétate de Na 10 mM, pH 4,0 et on les a appliquées sur une colonne Mono S (HR 5/5 Pharmacia) et éluées avec un gradient linéaire de NaCl (0 à 300 mM en 40 min) à une vitesse d'écoulement de 0,8 ml.min⁻¹. On a collecté la fraction contenant l'activité de la transférase, on l'a dialysée contre du Tris/HCl 10 mM, pH 7,0 et on l'a déposée sur une colonne DEAE-5PW (8 x 75 mm, TosoHaas) et on l'a éluée avec un gradient linéaire de NaCl (0 à 300 mM en 60 min) avec une vitesse d'écoulement de 0,75 ml.min⁻¹. On a collecté les fractions contenant l'activité de la transférase, on les a dialysées contre un tampon contenant du β-mercaptoéthanol 10 mM, de l'EDTA 5 mM, de l'acétate de Na 10 mM, pH 4,0 et on les a déposées sur une colonne CM-5PW (8 x 75 mm, TosoHaas) et on a élué avec un gradient linéaire de NaCl (0 à 300 mM en 60 min) avec une vitesse d'écoulement de 0,8 ml.min⁻¹. On a collecté les tractions contenant la transférase et on les a concentrées par un speed-vac et fractionnées sur une colonne superdex HR75 (Pharmacia) équilibrée avec du Tris/HCl 10 mM, pH 7,0 contenant du NaCl 150 mM, à une vitesse d'écoulement de 0,75 ml.min⁻¹. On a collecté les fractions contenant la transférase purifiée, dialysé contre du citrate de Na 5 mM, pH 5,0, concentré par speedvac et conservé à -20°C jusqu'à utilisation.

### 3. Dosages de la transférase

On a dosé les fractions d'enzymes pour la présence de l'activité de la transférase en incubant dans du citrate de Na 50 mM, pH 5.0 à 37°C (10 µl de volume de dosage) avec un laminarioligosaccharide réduit au borohydrure (8 mM final) d'au moins une taille G₁₀. On a pris des échantillons (3 µl) à des temps différents et ajouté du NaOH 50 mM refroidi dans de la glace (47 µl) pour terminer la réaction et on a congelé jusqu'à l'analyse par chromatographie échangeuse d'anions à haute performante (HPAEC). A cause des variations de l'intensité des pics de jour en jour avec une détection par Pulsed Electrochemical Detector (PED), on a quantifié l'activité de la transférase en utilisant des laminarioligosaccharides réduits marqués au ³H en tant que substrats et en mesurant l'apparition du marquage dans les produits après la séparation par chromatographie HPAEC, en utilisant un appareil en ligne 150TR d'analyse de vitesse de scintillation Radiomatic (Packard). A moins que cela ne soit mentionné, les dosages pour les études de la caractérisation des enzymes sont effectuées comme ci-dessus avec 0,25 µg de transférase purifiée.

### 4. Dosages colométriques

On a mesuré l'activité de le β-glucanase dans les fractions protéiques par un test réducteur à base de sucre en utilisant le réactif hydrazide de l'acide hydroxybenzoique avec de la laminarine réduite au borohydrure à la place de la carboxyméthyl pachymane an tant que substrat (Ram et al, 1988, Life sci Adv., 7, 379-383). On a mesuré les activités exo-β-glucanase/β-glucosidase en incubant les fractions d'enzymes avec du p-nitrophényl-β-D-glucopyranoside (Hartland et al., 1991, Proc. R. Soc. London B, 246, 155-160). On a estimé la quantité de protéines en utilisant le test de la protéine Biorad selon les instructions du fabricant, avec de l'albumine de sérum bovin comme standard.

### 5. Chromatographie échangeuse d'anions à haute performance.

On a analysé les échantillons provenant des tests de la transférase sur une colonne analytique Dionex CarboPac PA1 (4 x 250 mm) (avec une colonne de contrôle PA1) sur un système Dionex de type HPAEC avec une détection pulsée électrochimique (cellule PED-2), équipé avec une combinaison d'électrodes de référence pH-Ag/AgCl et en utilisant un potentiel de 0,4 V pour la première 0,5 s de détection. On a élué les oligosaccharides dans les conditions suivantes: vitesse d'écoulement de 1 ml/min, tampon A: NaOH 50 mM; tampon B: acétate de sodium 500 mM dans du NaOH 50 mM;gradient: 0 à 2 min, 98% A 2% B (isocratique), 2 à 15min 75% A 25% B (linéaire), 15 à 45 min 60% A 40% B (linéaire).

Les standards de laminarioligosaccharides ont été obtenus chez Seikagaku (Japon).

### 6. Chromatographie en couche mince (CCM)

On a visualisé les laminariologosaccharides par une chromatographie en couche mince sur un gel de silice 60 (Kieselgel, Merck) en utilisant du n-butanol/acide acétique/eau; (2/1/1,5) en tant qu'éluant et une coloration à base d'orcinol sulfurique.

On a également déterminé le degré de polymérisation (dp) des oligosaccharides par chromatographie de type HPAE en utilisant un détecteur pulsé électrochimique et une colonne échangeuse d'anions (Carbo6PAC PA1, 4,6 x 250 mm, Dionex).

### 7. Préparation des substrats réduits

On a obtenu les laminarioligosaccharides par une hydrolyse partielle acide (TFA 6,5 M, 15 min, 100°C, suivi par du TFA 1 M, 45 min, 100°C) de curdlan (Serva). On a éliminé le TFA par une évaporation rotatoire en présence de méthanol. On a réduit les oligosaccharides toute la nuit avec du NaBH₄ (1:0,5 (m/m) dans NaOH 0,1 M à température ambiante). On a préparé de manière similaire les extrémités réduites des laminarioligosaccharides marquées au ³H par réduction avec du NaB³H₄ (Amersham, 20-40 Ci/mmol, 10 mCi par mg d'oligosaccharides) toute la nuit suivi par une réduction supplémentaire avec du NaBH₄ comme avant. On a détruit l'excès de NaBH₄ en ajoutant de l'acide acétique jusqu'à un pH de 5-6, et on a éliminé les sels de borate par une évaporation rotatoire en présence de méthanol. On a dessalé les oligosaccharides réduits par une filtration sur gel sur une colonne de Sephadex G15 (1,2 x 80 cm, 8 ml.h⁻¹, équilibrée en eau) et on les a collecté après détection par la méthode à l'orcinol-acide sulfurique (Ashwell, 1966, Methods Enzymol, 8, 85-95). On a séparé les laminarioligosaccharides par HPAEC sur une colonne préparative CarboPac PA1 (9 x 250 mm) (Dionex) avec un gradient d'acétate de Na 15 à 350 mM dans du NaOH 50 mM (45 min) à une vitesse d'écoulement de 4 ml.min⁻¹. On a neutralisé les fractions collectées des oligosaccharides par de l'acide acétique, dessalé par filtration sur gel sur une colonne Sephadex G 15 comme décrit ci-dessus, puis hyophilisé. On a réduit de la même manière la laminarine (Sigma), mais on l'a dessalée par dialyse contre de l'acide acétique à 0,5%, suivi par une dialyse contre de l'eau, puis on a hyophilisé. On a préparé des gentioologosaccharides comme ci-dessus (sans réduction) à partir de pulsatine (Calbiochem) qui ont été finement divisé par un mortier et un pillon. Le maltoheptaose et le cellopentaose viennent de chez Boehringer Mannheim et Sigma, respectivement. La chitohexaose était un don généreux du Dr. A. Domard (Université Claude Bernard, Villeurbanne, France). On a obtenu le G₁₀ réduit au borohydrure contenant une liaison intrachaîne β-(1-6) à la sixième liaison à partir de l'extrémité réduite (rG₁₀*) en incubant le laminarihexose réduit (rG₆) avec une enzyme homologue à l'enzyme BGT1 de *Candida* purifiée à partir de *A. fumigatus.* Le produit de la transférase rG₁₀* a été séparé et purifié comme pour les laminarioligosaccharides ci-dessus.

### 7.Electrophorèse en gel de SDS-polyacrylamide

On a analysé des échantillons de protéines par SDS-PAGE (Laemmli, 1970, Nature, 227, 680-685) en utilisant des gels de séparation à 10% et des gels empillés à 4 %. On a visualisé des bandes de protéine en colorant au bleu de Coomassie. On a effectué La N-glycosylation des glycoprotéines en utilisant de la N-glucosidase F recombinante (Oxford GlycoSystems) selon les instructions du fabricant.

### 8. Spectroscopie RMN-¹H

On a analysé deux échantillons: le laminarioligosaccharide réduit G₁₀ utilisé comme standard et un oligosaccharide G₁₆ réduit qui a été obtenu après incubation du rG₁₀ avec la transférase et purifié par HPAEC. On a remplacé le deutérium dans les échantillons secs par hyophilisation en dissolvant dans D₂O (99,95%, Solvents Documentation Synthese, France). On a enregistré les spectres à 300 K et 318 K sur une spectrométrie 500 de type Variant Unity opérant à une fréquence des protons de 500 MHz. La résonance des OH de l'eau résiduelle a été supprimée par une rayonnement sélectif au cours du temps de relaxation. On a utilisé de l'acide 3-triméthylsilylpropionique de sodium comme témoin externe.

### EXEMPLE 1-Purification de la protéine de 49 Kda

Afin d'étudier les activités de la β-glucanosyl transférase associée à la paroi cellulaire dans *A. fumigatus,* on a développé un test de chromatographie échangeuse d'anions à haute performance (HPAEC) en utilisant des laminarioligosaccharides réduits au borohydrure comme substrats. On a détecté une nouvelle activité de la β-(1-3)-glucosyl transférase dans les fractions semi-purifiées provenant de l'autolysat de la paroi cellulaire de *A.fumigatus,* qui reste associée à une protéine de 49 kDa tout le long de sa purification.

On a purifié la protéine à une homogénéité apparente avec quatre étapes de chromatographie échangeuse d'ions et une étape de filtration sur gel.

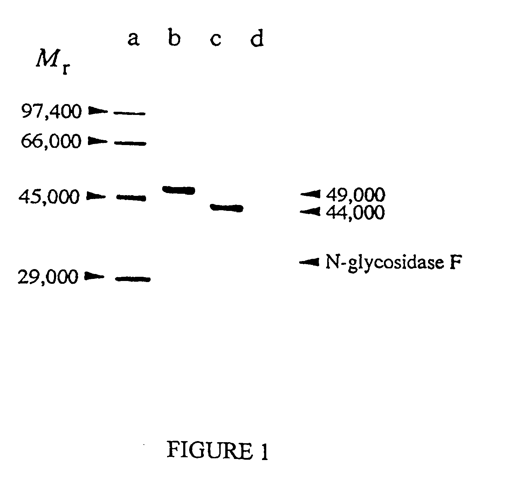

L'activité de la transférase était clairement détectable après seulement la seconde étape de chromatographie (Mono S). L'analyse par SDS-PAGE de la fraction purifiée a montré une bande principale à 49 kDa (fig. 1, puits b). Pour déterminer si elle contient un carbohydrate N-lié, on a digéré la protéine avec la N-glucosidase F. La protéine digérée est passée sur le SDS-PAGE comme étant une protéine de 44 kDa (fig. 1, puits c), indiquant qu'elle contient environ 5 kDa de carbohydrate N-lié.

### EXEMPLE 2: Activité enzymatique de la protéine de 49 kDa

L'analyse par HPAEC des produits résultant de l'incubation de la protéine de 49 kDa avec un laminarioligosaccharide réduit au borohydrure (rGₙ) de taille G₁₀ ou plus grand a permis la caractérisation d'une nouvelle activité de type glucanosyl transférase.

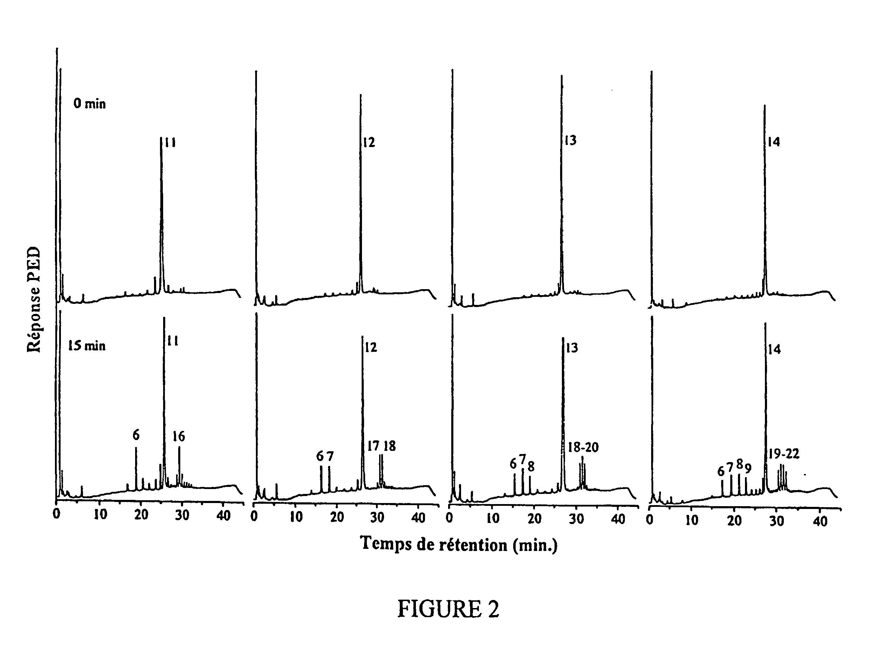

Les produits initiaux principaux provenant de l'incubation avec rG₁₁ étaient rG₆ et rG₁₆; rG₁₂ donnait rG₆ + rG₇ et rG₁₇ + rG₁₈, rG₁₃ donnait rG₆ à rG₈ et rG₁₈ à rG₂₀, et rG₁₄ résultait de la formation de rG₆ à rG₉ et rG₁₉ à rG₂₂ (figure 2). De manière importante, on n'a pas détecté de produits de type laminarioligosaccharide réduit ou non, confirmant l'absence de toute activité endo-β-(1-3)-glucanase. La présence d'une telle activité aurait engendré la formation d'un mélange de produits d'hydrolyse réduits ou non, le dernier ayant plutôt des temps de rétention différents. En plus, on n'a pas détecté de glucose, et ensemble avec l'absence de l'hydrolyse de la p-nitrophényl-β-glucopyranoside et la formation en réseau de sucre réducteur provenant de la laminarine réduite par le borohydrure dans les tests correspondant colorimétriques, on a confirmé l'absence de l'activité de l'exo-β-(1-3)-glucanase et β-glucosidase.

Le profil des produits obtenus (figure 2) est en accord avec une activité de la glucanosyl transférase de type endogène dans laquelle la chaîne de glucane est coupée par une coupure endolytique, libérant la portion de l'extrémité réduite, et le restant est transféré à une autre chaîne de glucane, formant un produit de type transférase plus grand. Ainsi, avec la plus simple réaction avec rG₁₁, l'enzyme coupe le substrat libérant rG₆, de l'extrémité réduite de la molécule du substrat, et le restant G₅ est alors transféré à une autre molécule rG₁₁ agissant comme récepteur, pour former le produit de type transférase rG₁₆:

E + rG₁₁ → E.G₅ + rG₆

E.G₅ + rG₁₁ → E + rG₁₆

où E représente l'enzyme. La transférase coupe rG₁₂ dans deux endroits différents engendrent deux produits différents de type transférase :

E = rG₁₂ → E.G₆ + rG₆ → E.G₅ + rG₇

E. G₆ + rG₁₂ → E + rG₁₈

E.G₅ + rG₁₂ → E + rG₁₇

De manière similaire avec rG₁₃ et rG_{14,} la transférase coupe dans trois ou quatre endroits différents, respectivement, chaque fois transférant la partie de l'extrémité non réduite à une autre molécule acceptrice rG₁₃ ou rG₁₄.

Des analyses supplémentaires d'incubations de la transférase de 49 kDa avec des laminarioligosaccharides réduits et plus petits ont montré que la réaction avec rG₁₀ donnait rG₅ + rG₆ et rG₁₄ + rG₁₅ comme produits initiaux majeurs, alors que la réaction avec rG₉ était extrêmement lente, formant des petits pics de rG₅ à rG₈ et rC₁₀ à rG₁₃. On n'a pas détecté de produits par incubation avec des laminarioligosaccharides réduits de taille G₈ et plus petit.

Pour déterminer la vitesse de la réaction relative des enzymes avec des laminarioligosaccharides de tailles variables, on a incubé l'enzyme de 49 kDa (0,25 µg) séparément avec 8 mM de rG₁₀ à rG₁₅ marqués au ³H et on a mesuré la vitesse de formation des produits marqués. La vitesse avec rG₁₀ (328 nmol.min⁻¹) était approximativement égale à 50% de celle avec des substrats plus gros et il n'apparaît aucune différence significative entre les vitesses de réaction pour rG₁₁ à rG₁₅ (648 ± 46 nmol.min⁻¹ .mg protéine⁻¹).

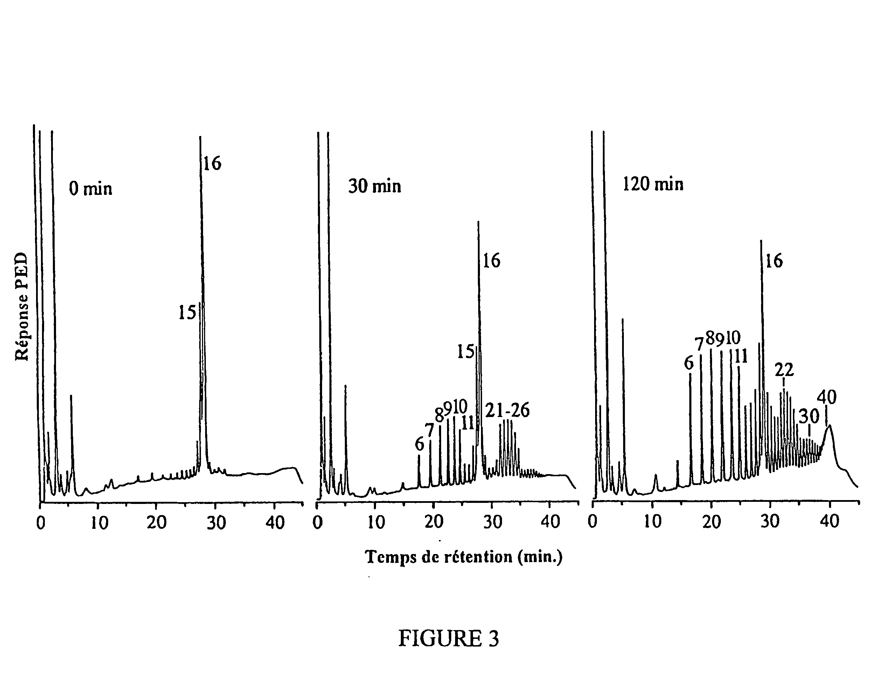

L'analyse d'incubations plus longues d'enzymes purifiées avec des laminarioligosaccharides réduits de taille au moins égale à G₁₀ a montré que les produits de la transférase initiale peuvent être ultérieurement ré-utilisés soit comme donneurs soit comme accepteurs, engendrant la formation de produits de taille croissante, jusqu'à ce qu'ils soient éliminés de la solution à cause de leur insolubilité dans le tampon aqueux. Une incubation de 30 min avec rG₁₆ (contenant certains contaminants rG₁₅) a engendré la formation de produits initiaux majeurs réduits, de tailles G₆ à G₁₁ et G₂₁ à G₂₆ (figure 3), mais après 120 min, des produits de la transférase plus gros sont apparus ayant jusqu'à au moins une taille égale à G₄₀ (figure 3). Les produits de taille G₂₉ et plus gros ont précipité dans le fond du tube d'incubation puisqu'ils manquaient lorsque le mélange réactionnel a été brièvement centrifugé et que le surnageant a été analysé, l'incubation de l'enzyme purifiée avec de la laminarine réduite a engendré la production de produits plus petits et plus gros, indiquant que les oligosaccharides solubles de taille égale à G₃₀ et plus gros peuvent agir en tant que donneurs et accepteurs dans la réaction.

Pour déterminer le plus petit laminarioligosaccharides qui puisse agir comme accepteur, la transférase purifiée a été incubée avec 4 mM de rG₁₁ comme donneur plus 16 mM de rG₈ ou plus petit comme accepteur. Les analyses des incubations contenant rG₁₁ plus rG₄ ou plus petit ont montré la formation de rG₆ et rG₁₆ comme seuls produits initiaux majeurs, indiquant que seul rG₁₁ a été utilisé comme accepteur. Cependant, des incubations contenant rG₁₁ plus rG₅ à rG₈ ont montré des produits additionnels de la transférase en conformité avec les derniers oligosaccharides ayant été utilisés comme accepteurs. Par exemple, la réaction de rG₁₁ plus rG₇ a engendré la formation initiale de rG₆, rG₁₂ et rG₁₆ en conformité avec:

E + rG₁₁ → E.G₅ + rG₆

E.G₅ + rG₁₁ →E+rG₁₆

E.G₅ + rG₇ → E + rG₁₂

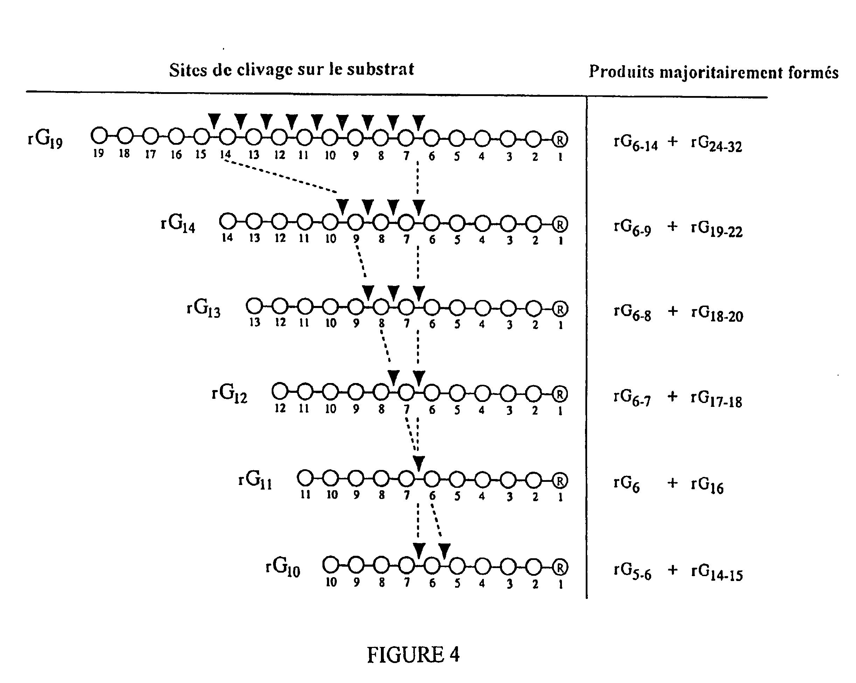

La vitesse relative de la réaction avec ces accepteurs a été déterminée en utilisant 2 mM de rG₁₁ comme donneur plus 32 mM d'accepteur réduit marqué au ³H et en mesurant la formation du produit marqué de la transférase (figure 4). Dans ces conditions, la réaction avec rG₁₁ utilisé comme accepteur est négligeable. La vitesse de la réaction augmentait avec l'augmentation de la longueur des chaînes, indiquant que l'enzyme de 49 kDa préfère des accepteurs de laminarioligosaccharides plus gros.

La transférase n'a montré aucune activité envers les gentiooligosaccharides (taille G₃₋₈), la chitohexaose, la cellopentaose ou la maltoheptaose, ni en présence ni en absence de rG₁₁, suggérant que l'enzyme utilisant exclusivement le β-(I-3) glucane comme un donneur a été démontré en utilisant un G₁₀ réduit branche (rG₁₀*) similaire de la laminaridécaose, sauf que la sixième liaison à partir de l'extrémité réduite est une liaison de type β-(1-6). L'incubation de l'enzyme de 49 kDa avec 8 mM de rG₁₀* n'a donné aucun produits indiquant que ce n'était pas un donneur. Cependant, une incubation similaire en présence de 2 mM de rG₁₁ a engendré la formation de rG₆ et un pic éluant en position de rG₁₅ comme produits initiaux majeurs, montrant que rG₁₀* peut agir comme accepteur.

### EXEMPLE 3: Analyse RMN-¹H du produit réduit G₁₆ de la transférase

Pour déterminer si la transférase de 49 kDa a produit un nouveau type de liaison au cours du transfert, le produit rG₁₆ de la transférase a été purifié du milieu d'incubation de la transférase avec rG₁₁. On a analysé approximativement 300 µg de produit par RMN-¹H. Le spectre 1D du produit rG₁₆ de la transférase présentait trois déplacements chimiques dans la région anomérique:
δ = 4,68 ppm correspondant au résidu de glucose lié au groupe glucitol;
δ = 4,75 ppm correspondant au résidu de glucose de l'extrémité non réduite;
δ = 4,80 ppm correspondant aux résidus intrachaîne de glucose lié en β-(1-3).

L'intensité relative des signaux anomériques indiquait 1,1 et 13 protons respectivement. Puisque le glucitol ne donne aucun signal dans la région anomérique, cela confirme la longueur de l'oligosaccharide (16 résidus). Les constantes de couplage mesurées sur ces signaux sont en accord avec les résidus de glucose liés en β (³J_{1,2} = 7,9 Hz). La présence d'un seul motif de type glucose à l'extrémité non réduite indiquait que la protéine de 49 kDa a été transférée sur l'extrémité non réduite de l'accepteur β-(1-3) glucane.

Le spectre 1D du produit rG₁₆ était identique, sauf pour l'intensité relative du signal de 4,80 ppm par rapport à celui du standard des laminarioligosaccharides rG₁₀. En plus, aucune caractéristique de déplacement chimique de résidu glucose lié en (1-2), (1-4), ou (1-6) n'a été vue confirmant que le produit rG₁₆ était une laminari-hexadécaose. L'élution conjointe du produit rG₁₆ avec la référence de rG₁₆ sur HPAEC et l'insolubilité du produit le plus gros sont en confirmité avec la production de liaison de type β-(1-3) au cours du transfert.

### Exemple 4: Effet de la concentration des substrats sur les produits de la réaction

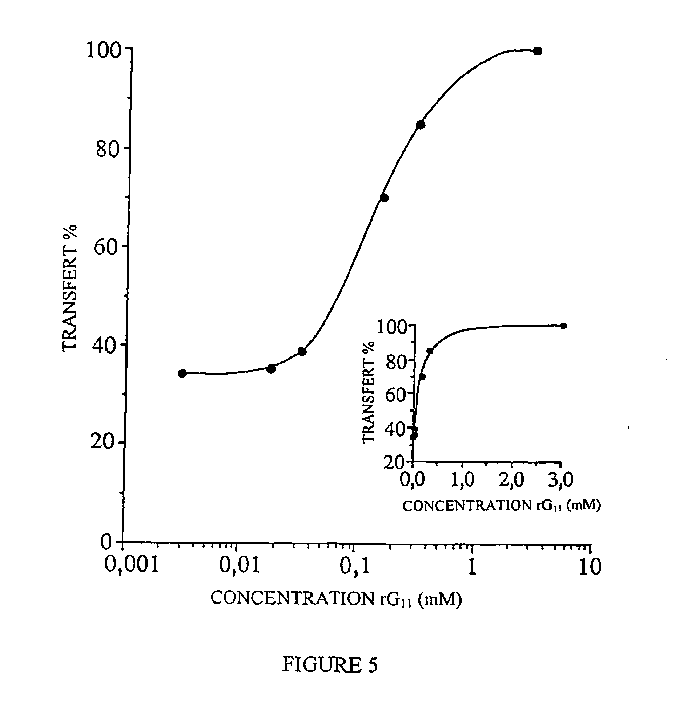

Pour déterminer si l'abaissement de la concentration des accepteurs stimulerait les réactions d'hydrolyse, on a incubé la transférase de 49 kDa avec 3 µl de [³H]-r-G₁₁ plus des quantités décroissantes de rG₁₁ non marqué. On a observé un déplacement du transfert (i) à l'hydrolyse (ii) (figure 5, supplément):

E + [³H]-rG₁₁ → E.G₅ +[³H]-rG₆

(i)

   E.G₅ + [³H)-rG₁₁ → E +[³H]-rG₁₆
(ii)

   E.G₅ + H₂O → E + G₅
On a déterminé le pourcentage de transfert en mesurant la formation de rG₁₆ marqué (seulement transfert) comparé à celle du rG₆ marqué (transfert plus hydrolyse) dans la réaction. A une concentration de rG₁₁ égale à 3 mM, seul le transfert a été détecté. Comme la concentration du substrat a baissé à 18 µM, le pourcentage de transfert s'est égalisé à environ 35% et n'a pas baissé de manière importante avec une faible concentration de substrat (3 µM) (figure 5). L'abaissement de la concentration du tampon à 10 mM n'a pas altéré le pourcentage du transfert à aucune des concentrations du substrat. Il apparaît qu'en dessous des conditions données, la transférase de 49 kDa était incapable de catalyser plus qu'environ 65% d'hydrolyse en simplement réduisant la concentration des substrat à des niveaux très faibles.

### EXEMPLE 5: pH optimum et stabilité.

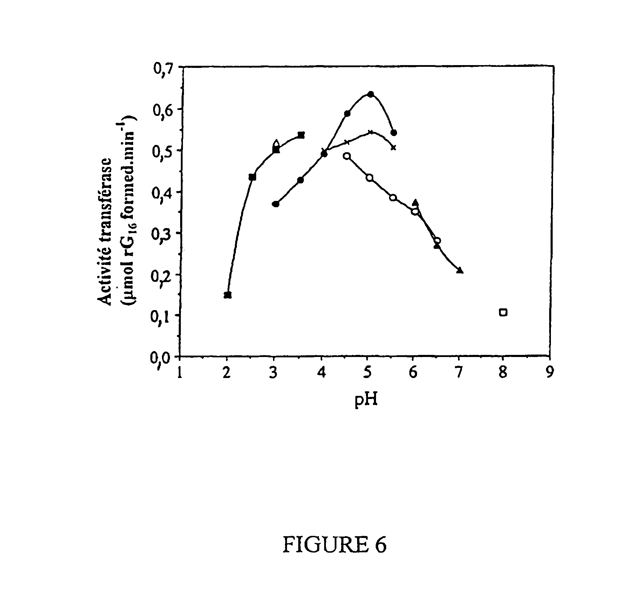

On a testé l'enzyme de 49 kDa à des valeurs de pH différentes, vérifié la stabilité au cours du stockage et on a testé l'activité de l'enzyme N-glycosylée. L'enzyme était active dans une large gamme de pH acide, montrant une activité supérieure à 50% du maximum entre pH 2,5 et 6,0. L'enzyme a manifiestée un pH optimum à environ 5,0 dans un tampon citrate (figure 6). L'enzyme était très stable et pouvait être stockée à 4°C dans un tampon citrate 10 mM, pH 5,0 pendant plusieurs semaines, ou séché par un speed-vac et ensuite remise en suspension dans un tampon, ou stockée à -20°C sans perte importante d'activité. L'enzyme de 44 kDa dé-N-glycosylée préparée dans des conditions non dénaturantes était aussi active que l'enzyme glycosylée native lorsqu'elle était incubée avec 8 mM de rG₁₁.

### EXEMPLE 6: Analyse de cinétique

L'enzyme de 49 kDa catalyse sa réaction de type transférase par un mécanisme de type bi-réaction (deux étapes) avec une hydrolyse initiale du substrat pour libérer la partie de l'extrémité réduite, et un transfert ultérieur du restant à l'extrémité non réduite d'une molécule de substrat jouant le rôle de molécule acceptrice.

En utilisant rG₁₁ en tant que substrat, il était impossible de calculer un Km apparent correspondant aux deux étapes de la réaction. Pour déterminer un Km pour le site donneur, nous avons utilisé un accepteur qui n'était pas un donneur. On a utilisé [³H]-rG₇(1x10⁶cpm) comme accepteur à une concentration élevée (64 mM) avec des concentrations variées de rG₁₁ gardées en dessous de 8 mM. Dans ces conditions, la réaction s'est effectuée avec rG₁₁ comme donneur et rG₇ était utilisé plus que rG₁₁ comme accepteur, comme cela est déterminé par l'absence de formation du produit de la transférase rG₁₆. La vitesse initiale de la réaction a été déterminée en mesurant l'apparition du rG₁₂ marqué.

On a obtenu un Km apparent de 5,3 mM à partir des doubles taches de type réciproque (valeur de r² = 0,997).

### EXEMPLE 7 : Clonage, séquençage et interruption du gène codant pour BUT2 dans A.fumigatus.

On a obtenu deux séquences d'acides aminés de la protéine BGT2 purifiée. La séquence NH₂ terminale était DVTPITVKGNAFFKGDERFY et une séquence interne était DAPNWDVDNDALP. Un oligonucléotide de 38 mer sur la partie N-terminale ayant la séquence suivante SEQ ID N°4:
(AAA GG(T/C) AA(C/T) GC(T/C) TTC TT(C/T) AAG GG(T/C) GA(T/C) GAG CG (T/C) TTC TA)
a été utilisé pour cribler une banque génomique construite dans le phage EMBL3 après une digestion partielle par Sau3A de l'ADN de *A.fumigatus* tel que décrit par Monod (1994, 33-40. Mol. Biology of pathogenic fungi, B. Maresca et G.S. Kobayashi).

Le transfert est effectué sur des membranes de type Zetaprobe. On a préhybridé et hybridé les membranes à 50°C dans une solution contenant du SSC 5X, Na2HPO4 20 mM, pH 7, SDS 7%, Denhardt 10X et du sperme de saumon à 1%. On a effectué le lavage des membranes à 42°C deux fois dans une solution contenant du SSC 3X, Denhardt 10X, SDS 5%, Na2HPO4 25 mM et deux fois dans une solution de SDS à 1% SSC 1X.

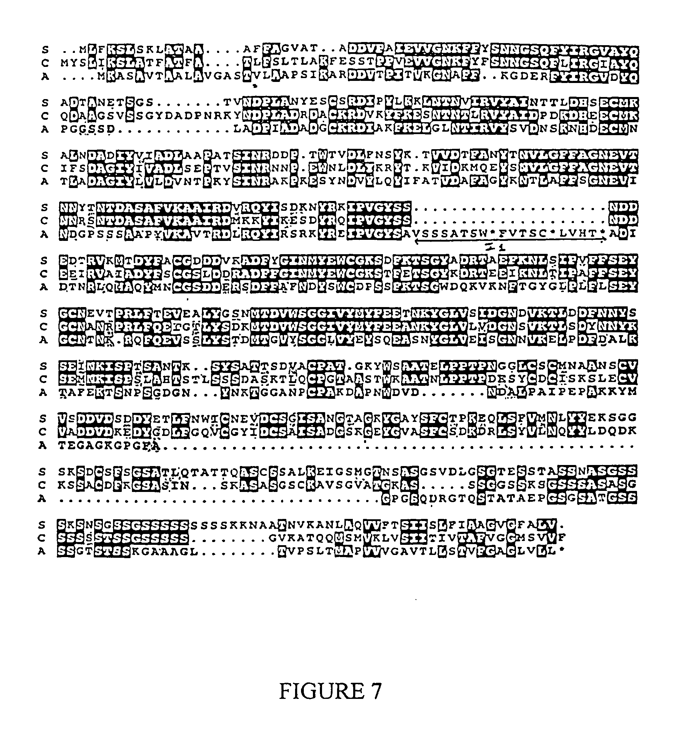

Le clonage et le séquençage du gène codant pour la protéine BGT2 a montré des homologies importantes avec les gènes PHR1 et GAS 1 identifiés auparavant dans *C.albicans* et *S.cerecisiae* respectivement (Saporito lrwin et Coll. (1995) Mol Cell Biol., 15, 601-613)(Nuoffer et Coll, J. Biol. Chem., (1991), 226, 19, 12242-12248) (figure 7). Le gène GAS 1 de *S.cerecisiae* est aussi responsable d'une activité glucanosyl transférase. Chez cette espèce de champignon, la taille minimale du substrat optimal est G10, au lieu de G11 pour *A.fumigatus.*

La dislocation du gène BGT2 a été effectué en utilisant le vecteur pAN7-1 (Punt et Coll. (1987) Gene 56, 117-124) fourni par P. Punt (TNO, Rijinsik), Ce vecteur a été modifié comme pN4 (Paris et Coll. (1993) FEMS Microbiol. Lett. 111, 31-36) par le remplacement d'un site de restriction HindIII par un site Sma 1. Environ 50% du cadre de lecture ouvert de BGT2 a été remplacé par pN4 à une site de restriction EcoRV. Une transformation intégrale a été faite comme décrit auparavant (Paris, (1994) Isolation of protease negative mutants of Aspergillus fumagitus by insertion of a disrupted gene, p. 49-55. Mol. Biology of pathogenic fungi, Maresca et G.S. Kobayashi) en utilisant des protoplastes produits par Novozyme et le plasmide linéarisé en présence de PEG.

Le mutant Δ 49 de *A.fumigatus* obtenu a été déposé le 30 juillet 1996 à la CNCM sous le numéro de dépôt I-1764.

Il ne présente aucun phénotype distinct de la souche sauvage, à l'exception d'une inhibition totale de la croissance en fermenteur après 24 heures de croissance.

### EXEMPLE 8 : clonage et séquençage de l'ADNc de BGT2

On a obtenu l'ADNc de BGT2 par amplification avec deux amorces de type ADNc 5' GAATTCGACGACGTTACTCCCATCACT 3' de P1 et 5'TCTAGAGGGTATGAGAAGAACAAATCA 3' de P2 provenant de 10 ng d'ADNc, 1U de taq polymérase, 200 mM de chaque amorces. On a effectué 30 cycles d'amplification, composés d'une minute à 95°C, une minute à 55°C et une minute à 72°C.

On a ensuite cloné la préparation amplifiée dans un vecteur avec l'aide d'un kit de Clonage TA (In Vitrogen).

### EXEMPLE 9 : Expression de la β(1-3)glucanosyltransférase

Des expériences utilisant des partages de Triton X114 avec ou sans traitement à la GPI-phospholipase C a montré que la protéine BGT2 de *A.fumigatus* est accrochée par un résidu GPI à la membrane plasmique.

L'accrochage de la protéine à la membrane n'est pas nécessaire pour conserver l'activité enzymatique. On a démontré que dans *A. fumigatus* la même activité était présente lorsque la protéine était soit libérée dans le milieu de culture (en absence de la liaison GPI) soit accrochée à la membrane plasmique.

Ces résultats suggèrent que l'expression de la glucanosyltransférase peut être faite dans des vecteurs par une expression sécrétée. Le système d'expression de *Pichia pastoris* par Invitrogen a été choisi. On a utilisé ce système auparavant avec une autre protéine de *A.fumigatus* de 88 kDa et il a été confirmé que Pichia conservait très bien le site de glycosylation de la protéine native.

Le vecteur utilisé est pPICZα (in Vitrogen) pour la sécrétion avec un épitope myc et six résidus histidine en tandem pour une purification facile. La séquence C-tenninale responsable de l'accrochage par GPI est éliminée avant le sous clonage dans pPICZα afin d'obtenir une protéine sécrétée tronquée active du point de vue enzymatique.

Cette protéine recombinante est utilisée pour la détection de médicaments antifongiques. L'inhibition de l'activité enzymatique peut être mesurée par une détection de type HPLC en absence de clivage et une élongation supplémentaire de tout β-(1-3) laminarioligosaccharides de dp>= 10 en présence de p49 de *A.fumigatus.*

L'absence de motilité du substrat laminariologosaccharide mesuré par une chromatographie en couche mince visualisé par des techniques classiques ou directement après le marquage de l'extrémité réduite avec un radical chromogène ou fluorogène peut être une technique de base pour effectuer une détection automatisée.

Puisque le produit de la β-(1-3)glucanosyltransférase devient insoluble dans un milieu aqueux, du fait de l'élongation de la chaîne β-glucan, l'absence de précipitation de tout produit après une incubation prolongée en utilisant un substrat marqué de façon radiocative peut également être utilisé pour la détection de médicaments.

### EXEMPLE 10 Identification de gènes présentant des homologies avecBGT2

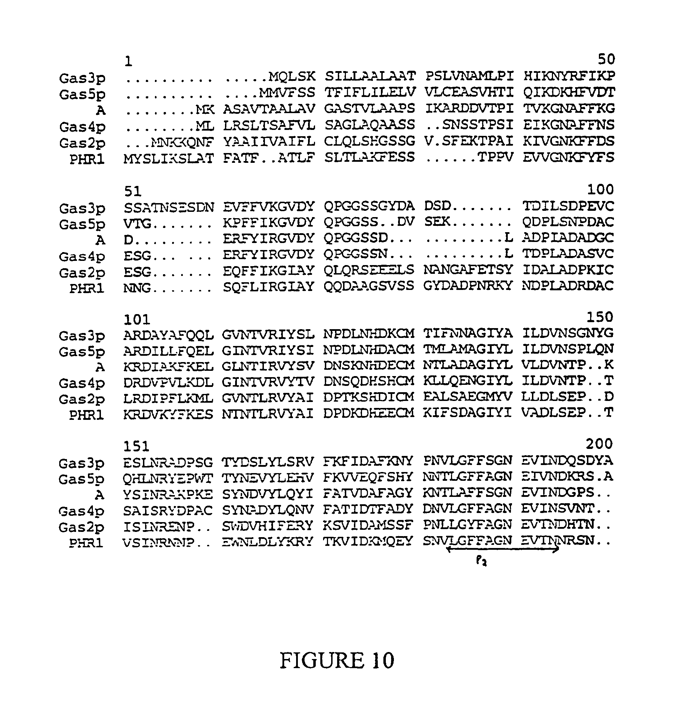

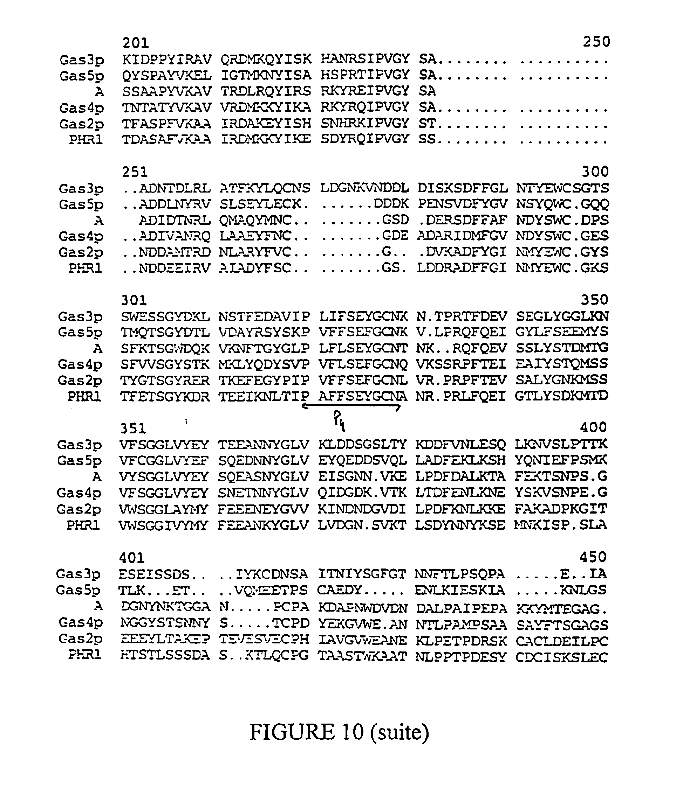

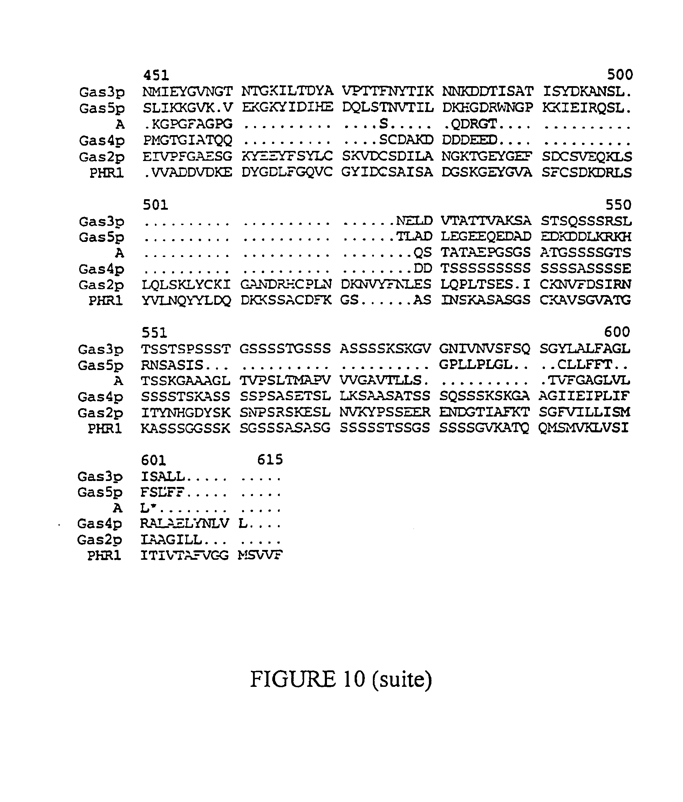

Des amorces oligonucléotidiques dégénérées correspondant aux régions conservées de la séquence de la figure 10 ont été synthétisées par la société GENSET. Elles présentent les séquences suivantes :
SEQ ID N°7 (P3) : 5' GSYTTCTTCKCYGGCAACGAGGTT 3'
SEQ ID N°8 (P4') : 5' GTTGCAGCCGWATTCGGASAYGAA 3'
dans lesquelles Y est C ou T
K est T ou G
S est C ou G
W est A ou T

Les réactions PCR ont été mises en oeuvre dans un volume de 100 µl contenant 1,5 mM MgC12, 50 mM Kcl, 10 mM TrisCl (pH8), 250 µM de dATP, dGTP, dCTP et d TTP (Pharmacia), 1 µM de chaque amorce, 2,5 unités d'Amplitaq DNA polymérase (Pharmacia) et 50 ng d'ADN génomique. L'amplification a été effectuée dans un appareil (OmniGene) tout d'abord durant 5 min à 93°C, puis durant 30 cycles de 1 min à 93°C, puis de 1 min à 50 °C et de 1 min à 72°C. Les produits de la réaction PCR ont été analysés par électrophorèse sur un gel d'agarose à 1 % puis visualisés à l'aide des ultraviolets après coloration par du bromure d'éthidium.

Les fragments résultant de la réaction PCR ont été ligaturés dans pCR2.1 (TA cloning kit, In Vitrogen). Les inserts des plasmides recombinants ont été séquencés par la méthode de terminaison de chaîne à la didéoxy (Sanger et al, 1977) en utilisant la Sequenase, Version 2 (US Biochemicals) conformément aux instructions du fabricant.

Deux séquences nucléotidiques ont été ainsi amplifiées : les séquences SEQ ID N°9 et SEQ ID N°11. Les séquences d'acides aminés déduites des séquences nucléotidiques sont les séquences SEQ ID N°10 et SEQ ID N°12, correspondant aux gènes appelés BGT4 et BGT3.

Ces séquences sont en particulier décrites dans la liste ci-après. Elles présentent des pourcentages d'identité avec BGT2 respectivement de 41 % et 37%.

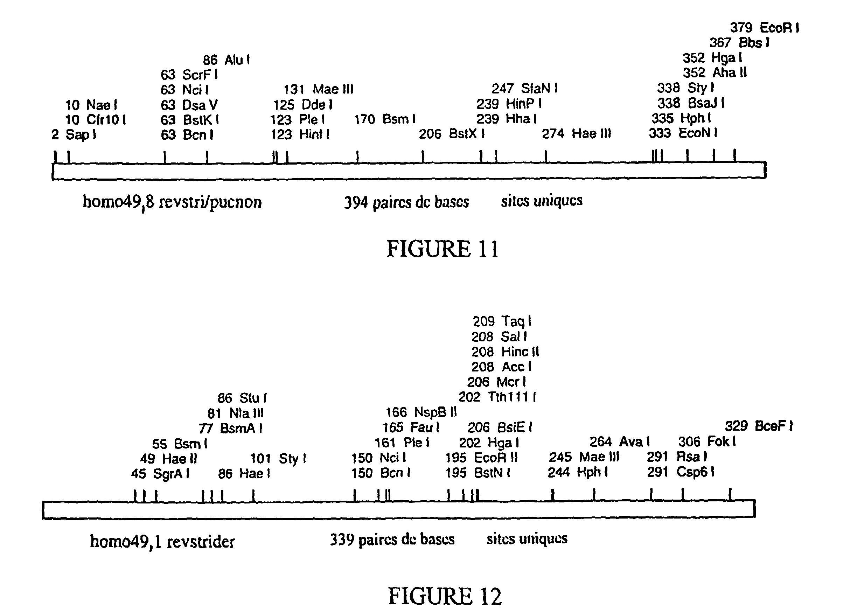

Les cartes de restriction des deux séquences SEQ ID N°10 et SEQ ID N°12 sont représentées respectivement sur les figures 12 et 11.

Les séquences SEQ ID N°9 et N°11 ont été insérées dans le plasmide PCRllqui a été introduit dans E. Coli. Ces bactéries ont été déposées le 22 août 1997 auprès de la CNCM, respectivement sous les n°I-1914 et I-1913.

### TABLEAU

### Réaction de la transférase avec une vitesse initiale de formation du produit de transfert

| Réaction de la transférase | Vitesse de transfert (nmol.min⁻¹.mg protéine⁻¹) |
|---|---|
| rG₁₁ + [³H]-rG₅ → RG₆ +[³H]-rG₁₀ | **203** |
| rG₁₁ + [³H]-rG₆ → RG₆ +CH]-rG₁₁ | **387** |
| rG₁₁ + [³H]-rG₇ → RG₆ +[³H]-rG₁₂ | **484** |
| rG₁₁ + [³H]-rG₈ → RG₆ +[³H]-rG₁₃ | **586** |

## Revendications

1. Protéine ayant une activité de type β(1-3) glucanosyltransférase **caractérisée en ce qu'**elle présente au moins 85% d'identité avec la protéine ayant une des séquences SEQ ID N°2 ou SEQ ID N°3 suivantes :

2. Protéine non glycosylée de séquence SEQ ID N°2 selon la revendication 1, **caractérisée en ce qu'**elle présente un poids moléculaire de 44 kD.

3. Protéine selon la revendication 1, **caractérisée en ce qu'**elle porte un radical N-glycosyle.

4. Protéine glycosylée de séquence SEQ ID N°3, **caractérisée en ce qu'**elle présente un poids moléculaire de 49 kD.

5. Protéine selon l'une des revendications 3 et 4, **caractérisée en ce qu'**elle est ancrée sur la membrane par un radical glycosylphosphatidylinoskol.

6. Souche *d'E.coli* déposée auprès de la CNCM sous le numéro de dépôt I-1762 portant la séquence SEQ ID N°1.

7. Souche *d'E.coli* déposée auprès de la CNCM sous le numéro de dépôt I-1763 portant la séquence complémentaire d'ADN capable de coder pour une protéine de séquence SEQ ID N°2.

8. Méthode pour la détection de mutations dans la séquence SEQ ID N°1, dans un échantillon biologique contenant des séquences nucléotidiques, comprenant les étapes suivantes :
a) mise en contact de l'échantillon biologique avec les amorces nucléotidiques P3 et P4 présentant respectivement les séquences SEQ ID N°7 et SEQ ID N°8 suivantes :
SEQ ID N°7 :
GSYTTCTTCK CYGGCAACGA GGTT
SEQ ID N°8;
GTTGCAGCCG WATTCGGASAYGAA
les sequences nucléotidiques contenues dans l'échantillon ayant été éventuellement mises sous une forme permettant leur hybridation dans des conditions permettant l'hybridation des amorces avec les séquences nucléotidiques ;
b) amplification de séquences nucléotidiques,
c) mise en évidence des produits d'amplification, et
d) détection des mutations vis-à-vis de la séquence SEQ ID N°1, par des techniques appropriées.

9. Procédé pour la détection de l'activité antifongique de molécules, par inhibition de l'activité de la β-(1,3)glucanosyltransférase, comprenant les étapes suivantes :
- mettre en présence la molécule à détecter et une protéine selon la revendication 4, ou codée par la séquence SEQ ID N°1, et
- déterminer l'effet inhibiteur d'une telle molécule sur ladite protéine, en mesurant l'activité de type β(1-3)glucanosyltransférase de ladite protéine.

10. Procédé selon la revendication 9 **caractérisé en ce que** la β(1-3)glucanosyltransférase est déterminée en mettant en présence ladite protéine et un substrat de β(1-3)glucanosyltransférase.

11. Procédé selon la revendication 10, **caractérisé en ce que** ledit substrat est composé au moins d'un laminarioligosaccharide comprenant au moins 10 radicaux glucosyl.

12. Procédé selon la revendication 11 **caractérisé en ce que** l'activité de la protéine sur son substrat est mise en évidence par chromatographie, en particulier par HPLC ou CCM, ou par toute méthode permettant la séparation d'oligosaccharides ayant des degrés différents de polymérisation.

## Claims

1. Protein having an activity of the β(1-3)-glucanosyltransferase type **characterized in that** it has at least 85% of homology with the protein having one of the sequences SEQ ID N°2 or SEQ ID N°3 as follows:

2. Non-glycosylated protein having the sequence SEQ ID N°2 according to claim 1 **characterized in that** it has a molecular weight of 44 kD.

3. Protein according to claim 1 **characterized in that** it carries an N-glycosyl radical.

4. Glycosylated protein having the sequence SEQ ID N°3 **characterized in that** it has a molecular weight of 49 kD.

5. Protein according to any of claims 3 and 4 **characterized in that** it is attached to the membrane by a glycosylphosphatidylinositol radical.

6. *E. coli* strain deposited at the CNCM under the deposit number I-1762 carrying the sequence SEQ ID N° 1.

7. *E. coli* strain deposited at the CNCM under the deposit number I-1763 carrying a complementary DNA sequence able to code for a protein of SEQ ID N°2.

8. Method for detecting mutations of the sequence SED ID N°1, in a biological sample containing nucleotide sequences, comprising the following steps:
a) placing the biological sample in contact with the nucleotide primers P3 and P4 having the sequences SEQ ID N° 7 and SEQ ID N° 8, respectively, as follows:
SEQ ID N° 7:
GSYTTCTTCKCYGGCAACGAGGTT
SEQ ID N°8:
GTTGCAGCCGWATTCGGASAYGAA:
the nucleotide sequences contained in the sample having been optionally put into a form enabling their hybridization under conditions enabling the hybridization of the primers with the nucleotide sequences;
b) amplificating the nucleotide sequences
c) revealing the amplification products, and
d) detecting the mutations in relation with the sequence SEQ ID N°1 by appropriate methods.

9. Process for detecting the antifungal activity of molecules by the inhibition of the β(1-3)-glucanosyltransferase activity comprising the following steps:
- placing the molecule to be detected in contact of a protein according to claim 4, or coded by the sequence SEQ ID N°1, and
- determining the inhibition effect of such molecule on said protein by measuring the β(1-3)-glucanosyltransferase activity of such protein.

10. Process according to claim 9 **characterized in that** the β-(1-3) glucanosyltransferase is determined by placing said protein in the presence of a substrate of β-(1-3) glucanosyltransferase.

11. Process according to claim 10 **characterized in that** said substrate is composed of at least one laminarioligosaccharide comprising at least 10 glucosyl radicals.

12. Process according to claim 11 **characterized in that** the activity of the protein on its substrate is detected by chromatography, in particular by HPLC or TLC, or by any method which enables the separation of oligosaccharides having different degrees of polymerization.

## Patentansprüche

1. Protein mit einer Aktivität des Typs β-(1-3)-Glucanosyl-Transferase, **dadurch gekennzeichnet, dass** es wenigstens 85% Identität mit dem Protein aufweist, das einer der folgenden Sequenzen SEQ ID Nr. 2 oder SEQ ID Nr. 3 aufweist:

2. Nichtglycosyliertes Protein der Sequenz SEQ ID Nr. 2 gemäß Anspruch 1, **dadurch gekennzeichnet, dass** es ein Molekulargewicht von 44 kD aufweist.

3. Protein gemäß Anspruch 1, **dadurch gekennzeichnet, dass** es einen N-Glycosylrest trägt.

4. Glycosyliertes Protein der Sequenz SEQ ID Nr. 3, **dadurch gekennzeichnet, dass** es ein Molekulargewicht von 49 kD aufweist.

5. Protein gemäß einem der Ansprüche 3 und 4, **dadurch gekennzeichnet, dass** es über einen Glycosylphosphatidylinosit-Rest auf der Membran verankert ist.

6. *E.-coli*-Stamm, der unter der Hinterlegungsnummer I-1762 bei der CNCM hinterlegt ist und die Sequenz SEQ ID Nr. 1 trägt.

7. *E.-coli*-Stamm, der unter der Hinterlegungsnummer 1-1763 bei der CNCM hinterlegt ist und die komplementäre DNA-Sequenz trägt, die für ein Protein der Sequenz SEQ ID Nr. 2 codieren kann.

8. Verfahren zum Nachweis von Mutationen in der Sequenz SEQ ID Nr. 1 in einer biologischen Probe, die Nucleotidsequenzen enthält, umfassend die folgenden Schritte:
a) In-Kontakt-Bringen der biologischen Probe mit den Nucleotidprimern P3 und P4, die die folgenden Sequenzen SEQ ID Nr. 7 bzw. SEQ ID Nr. 8 aufweisen:
SEQ ID Nr. 7:
**GSYTTCTTCK CYGGCAACGA GGTT**
SEQ ID Nr. 8:
**GTTGCAGCCG WATTCGGASAYGAA**
wobei die in der Probe enthaltenen Nucleotidsequenzen gegebenenfalls in eine Form gebracht wurden, die ihre Hybridisierung ermöglicht, unter Bedingungen, die die Hybridisierung der Primer mit den Nucleotidsequenzen erlauben;
b) Amplifikation von Nucleotidsequenzen;
c) Nachweisen der Amplifikationsprodukte; und
d) Nachweis der Mutationen gegenüber der Sequenz SEQ ID Nr. 1 durch geeignete Techniken.

9. Verfahren zum Nachweis der fungiziden Wirkung von Molekülen durch Hemmung der Aktivität der β-(1-3)-Glucanosyl-Transferase, umfassend die folgenden Schritte:
- Bereitstellen des dem Nachweis zu unterziehenden Moleküls und eines Proteins gemäß Anspruch 4 oder eines Proteins, das durch die Sequenz SEQ ID Nr. 1 codiert wird; und
- Bestimmen der hemmenden Wirkung eines solchen Moleküls auf das Protein, indem man die Aktivität des Typs β-(1-3)-GlucanosylTransferase des Proteins misst.

10. Verfahren gemäß Anspruch 9, **dadurch gekennzeichnet, dass** die β-(1-3)-Glucanosyl-Transferase **dadurch** bestimmt wird, dass man das Protein und ein Substrat der β-(1-3)-Glucanosyl-Transferase bereitstellt.

11. Verfahren gemäß Anspruch 10, **dadurch gekennzeichnet, dass** das Substrat aus wenigstens einem Laminarioligosaccharid besteht, das wenigstens 10 Glucosylreste umfasst.

12. Verfahren gemäß Anspruch 11, **dadurch gekennzeichnet, dass** die Wirkung des Proteins auf sein Substrat durch Chromatographie, insbesondere HPLC oder DC, oder durch irgendein anderes Verfahren, das die Trennung von Oligosacchariden mit unterschiedlichen Polymerisationsgraden erlaubt, nachgewiesen wird.
